# EUROPEAN PATENT APPLICATION

(11) **EP 0 704 209 A1**
(43) Date of publication of application: **03.04.1996**
(21) Application number: 95306653.7
(22) Date of filing: 20.09.1995
(51) Int. Cl.: A61K 31/195, A61K 9/107, A61K 9/06

(54) **Transdermally active pharmaceutical composition containing 5-aminolaevulinic acid**

(30) Priority: 20.09.1994 US 309296
(71) Applicant: JOHNSON & JOHNSON MEDICAL, INC., Arlington, Texas 76004-3030 (US)
(72) Inventor: Liu, Jue-Chen, Neshanic, NJ 08853 (US); Yusuf, Mohammed, Edison, NJ 08820 (US); Huddleston, Catherine, Somerville, NJ 08876 (US); Wang, Jonas C.T., Robbinsville, NJ 08691 (US)
(74) Representative: Fisher, Adrian John

(57) **Abstract**

This invention relates to a ready-to-use, storage stable composition for use in the treatment of cancer which contains 5-aminolaevulinic acid or its derivatives and has a pH from about 1.8 to about 4.5.

## Description

### 1. Field of the Invention

This invention relates to a transdermally-active pharmaceutical composition containing phototoxically active compounds for the treatment of cancer. More particularly, this invention relates to a pharmaceutical composition, method for making said composition and a method of using said composition containing 5-aminolaevulinic acid or a phototoxically active derivative thereof. The compositions of this invention are medically acceptable to the patient when applied topically to the skin and are physically and chemically stable in storage.

### 2. Prior Art

Since the investigation of H. Auler and G. Sanzer, ("Studies on the Role of Porphyrin in Human and Animal Tumors", Z. Krebsforsch. 53, 65-68 (1942)), it has been known that haematoporphyrins are absorbed particularly well by neoplastic tissue. R.L. Lipson et al., (Cancer, Proc. IX. Internat. Cancer Congr. 1966, 393) describe the use of haematoporphyrin derivatives for the destruction of tumor cells by means of radiation and light. This destructive action of haematoporphyrin and light on tumor cells has been confirmed by numerous authors. Since the investigations of N.I. Berlin et al. (Biochem. J. 64 (1956), 80-100), it has been known that a 5-aminolaevulinic acid is converted into porphyrin by the human metabolism.

For many years, these findings have served as a basis for the treatment of tumor patients, whereby the active compounds have been administered orally as well as by means of injections. However, these two types of systemic administration have the disadvantage that the body converts the aminolaevulinic acid ("ALA") into a photosensitive porphyrin derivative within the entire body. This renders the patient photosensitive, which requires that the whole skin of the patient must be protected from light radiation. This photosensitivity can last for between two weeks and four months, during which the patient must be protected from any sunlight. The patient, therefore, is usually kept in darkened rooms until the active compounds have been completely metabolized into light-insensitive substances. This treatment, therefore, is extremely inconvenient for the patient. A satisfactory solution to this problem has not been available heretofore, despite intensive efforts in the art.

WO 91/01727, and in the publication of Kennedy et al in J. Photochem. Photobiol., B: Biology 1990: 143-148, the authors proposed adding the active compound as a topical application of 20% ALA dissolved in Glaxal Base to the tissue to be treated three to six hours before exposure. The formulation was freshly prepared prior to each application and use.

R. M. Szeimies et al., (Photochemistry and Photobiology, Vol. 59, pp. 73-76 (1994))describe the penetration potency topically applied 5-ALA for photodynamic therapy of basal cell carcinoma. A composition containing 10% ALA and 10% highly dispersed SiO₂ in propylene glycol was prepared fresh prior to use. The propylene glycol was used as a solvent and the highly dispersed SiO₂ was used to increase the viscosity of the composition. However, this composition did not exhibit an acceptable penetration rate. Up to twelve (12) hours of uptake was required to detect a homogeneous fluorescence in the tumor areas.

Svanberg, et al., "Photodynamic therapy of non-melanoma malignant tumors of the skin using topical δ-amino levulinic acid sensitization and laser irradiation", British J. Derm. (1994) 130, p. 743-751 describes a cream containing ALA which was prepared fresh prior to application. We found this formulation to have a pH of between 1.4 and 1.7.

It has also been shown that a mere topical application can only treat neoplasms that are not thicker than three (3) millimeters, because the active compound does not penetrate deeper into the skin layer.

This invention, therefore, is intended to provide a pharmaceutical composition or formulation which can be prepared prior to use, i.e., is ready to use, rather than be prepared fresh for each use. It is another object of this invention to provide a pharmaceutical composition which is chemically and physically stable in storage and will have a shelf life sufficient for industrial production and clinical application. It is another object of this invention to provide a pharmaceutical composition which can be applied topically, having a higher penetration depth and a higher passive penetration rate than those compositions heretofore known in the art.

### BRIEF DESCRIPTION OF THE DRAWINGS

Figure 1 depicts a Franz diffusion cell for use in measuring skin permeation of a drug.

Figure 2 is a graph depicting results of a skin permeation test using the Lund (Svanberg et al.) formulation and the formulation of Example 3.

### SUMMARY OF THE INVENTION

In accordance with the teachings of this invention, the objects set forth above may be achieved using a pharmaceutical, ready-to-use composition containing 5-aminolaevulinic acid or a derivative thereof and having a pH of about 4.5 or below. This composition is medically acceptable to be applied topically to the skin and is physically and chemically stable in storage. The inventors have found that, not only are the compositions of this invention chemically and physically stable, but, surprisingly, they are tolerated by patients' skin, notwithstanding the acidity of the compositions.

### DETAILED DESCRIPTION OF THE PREFERRED EMBODIMENTS

Preferably, the compositions of this invention have a pH of about 4.5 or lower. More preferably, the pH should be from about 1.8 to about 3.6. The pH should not be lower than about 0.9, preferably, it should not be lower than about 1.8. Most preferably, the pH should be from about 1.8 to about 3.1. In this range of pH, the irritation level of the compositions is low, the level of skin permeation is high and the composition is storage-stable.

Preferably, the composition should be made in the form of a cream or an ointment or other form known to those of ordinary skill in the art. Among different types of formulations, such as gels, milks, lotions, liposome suspensions and oil-in-water emulsions, the more preferable have been oil-in-water emulsions. The oil-in-water emulsions have proven to be chemically and physically stable and are pharmaceutically acceptable at low, acidic pH's of the composition of the invention. Furthermore, the oil-in-water emulsions of the compositions of this invention provide a deeper and faster penetration of the preferred active agent, 5-aminolaevulinic acid.

Preferably, the hydrophobic oil/fat material contained in the preferred oil-in-water emulsions of this invention are hydrocarbons, more preferably, aliphatic hydrocarbons. On example of such materials are paraffin, which can be solid or fluid. Preferably, the paraffin is in the form of vaseline or paraffin liquid or white paraffin, in particular, mineral oil and/or petrolatum or the like.

The compositions of this invention should, preferably, contain an emulsifier. The emulsifier may be anionic, cationic, amphoteric and/or nonionic. The emulsifiers useful in the compositions of this invention should contain lipophilic groups such as alkyl radicals having from about 6 to about 22 carbon atoms. These alkyl radicals may be saturated or unsaturated. Further, these alkyl radicals may be linear or branched. The alkyl radicals useful as lipophilic groups in the compositions of this invention may also include aromatic radicals or alkylaryl radicals.

The emulsifiers useful in the compositions of this invention also generally include one or more hydrophilic groups. Examples of such hydrophilic groups include carboxylates, sulfonates, sulfates, phosphates, polyphosphates, lactates, citrates and/or tartrates and the like.

Cationic emulsifiers useful in the compositions of this invention include amine salts and/or quaternary ammonium compounds. Amphoteric emulsifiers may include groups such as NH₃⁺, --COO-, or the respective betaine structure. Examples of nonionic emulsifiers useful in the compositions of this invention are alcohols, polyethers, glycerol radicals, sorbitol radicals and radicals such as pentaerythrites, saccharose, acetic acid and lactic acid. If desired, such emulsifiers may also contain hydrophilic units in the middle of the molecule, for example, esters, polyamines, polyamides or polyethers.

Preferably, a mixture of alkyl alcohols, for example, cetyl alcohol and stearyl alcohol are used. Preferably, such a mixture should contain minor amounts of an anionic alkylate such as fatty alcohol sulfates with a carbon backbone having from about 14 to about 18 carbon atoms. More preferably, cetyl sulfate and stearic sulfate are employed; most preferably, the alkali salts, preferably the sodium salts, are used. For example, ceto-stearyl alcohol is useful in the compositions of this invention.

More preferably, nonionic surfactants based upon polyethylene glycol ethers of lauryl, cetyl, stearyl and/or oleyl alcohols are useful as emulsifiers in the compositions of this invention. Preferred are the noniinic surfactants available from the Atlas Chemie company under the brand name Brij (e.g., Brij 58). Preferably, the nonionic surfactants make up from about 1 to about 5% by weight of the total composition.

In addition, the compositions of this invention may also preferably contain Cetomacrogol 1000, an emulsifier which is generically known as polyoxyethylene-20 cetyl ether, in the amount of from about 0.5 to about 5 weight percent.

The compositions of this invention may also contain thickening agents, which may be natural thickeners or derivatives thereof. For example, alginates, pectins or carboxy methyl cellulose and other cellulosic ethers may be utilized. Other thickening agents which can be included in the compositions of this invention are synthetic thickeners, such as polyacryl and polymethacryl compounds, polyvinylic polymers, polycarboxylic acids and polyethers. Especially preferred are inorganic thickeners, such as dispersed silica, polysilicates and clay minerals such as montmorillonite, zeolite and phyllosilicates.

Preferably, the compositions of this invention include a penetration enhancer that improves the permeability of the skin with respect to the active compound. Examples of such penetration enhancers include decyl methyl sulfoxide (DeMSO), enhancing surfactants, such as sodium lauryl sulfate or its derivatives or other enhancers, as acetyl cysteine compounds and acid-stable liposomes and the like.

The compositions of this invention may also include preservatives such as chlorocresol, parabenes and/or phenoxy ethanol or the like in order to keep the compositions relatively free of bacteria.

If not otherwise specified, all the percentages given herein are by weight.

Preferably, the compositions of this invention contain from about 2 to about 40% by weight of 5-aminolaevulinic acid or a derivative thereof. More preferably, they contain from about 10 to about 30% by weight of 5-aminolaevulinic acid. Preferably, said compositions contain from about 5 to about 50% of a fat, wax and/or oil, from about 0 to about 10% by weight of an emulsifier, from about 0 to about 10% by weight of a penetration enhancer and, if desired, up to about 1% by weight of a preservative.

Especially preferred is a composition according to this invention containing from about 5 to about 15% by weight of petrolatum, from about 0.1 to about 10% by weight of mineral oil, from about 0 to about 5% by weight of cetyl alcohol, from about 0 to about 5% by weight of stearyl alcohol, from about 1 to about 5% by weight of Brij 58, from about 0.01 to about 0.06% chlorocresol, from about 0 to about 0.3% by weight of phosphoric acid (85%) and from about 5 to about 30% by weight of 5-aminolaevulinic acid, the remainder being water.

In another preferred embodiment, the composition of the invention contains about 20% by weight of water, about 20% by weight of 5-aminolaevulinic acid, and about 50% by weight of a cream containing 9% vaseline, 3.6% of white paraffin (paraffinum liquidum), 4.32% by weight of cetostearyl alcohol, 1.35% by weight of Cetomacrogol 1000, 0.06% by weight of chlorocresol, 0.18% of sodium dihydrogen phosphate dihydrate and 0.0012% of concentrated phosphoric acid (85%), the remainder being purified water. Preferably, the pH is about 3.

This invention further relates to a process for preparing the pharmaceutical compositions of this invention. According to the process of this invention, a cream or ointment is prepared by adding together water and oil, fat and/or wax and the active agent 5-aminolaevulinic acid, together with desired additional excipients and additives. From this mixture an emulsion is prepared in a manner known to those of ordinary skill in the art. In contrast with the traditional procedure of preparing such compositions, wherein a cream or ointment base is prepared first, then an aqueous solution of the active ingredient, which is then added to the cream base, the composition of this invention is prepared as follows: the cream or ointment base is degassed and/or kept under an inert atmosphere (using argon, nitrogen or helium gas). The aminolaevulinic acid-containing solution is intimately mixed with the cream, said mixing being carried out under an inert atmosphere, e.g. by purging with an inert gas. The final product is then transferred into a light-impermeable, acid-resistant, flexible container, which is preferably air-tight.

This invention also relates to the use of the compositions of this invention in the treatment of cancer in animals, particularly in humans. The compositions of this invention may be applied topically and may be driven into the skin using techniques known to those of skill in the art, such as iontopheresis, phonopheresis and the like.

The following examples serve to illustrate, but not limit, the scope of this invention.

### Example 1

20 g of water and 20 g of 5-aminolaevulinic acid were added to a 500 ml beaker and stirred slowly at room temperature until the solution became clear. The solution was then degassed and kept under a nitrogen atmosphere. 60 g of a cream containing 9 g vaselinum album, 3.6 g paraffinum liquidum, 4.32 g cetostrearyl alcohol, 1.35 g Cetomacrogol 1000, 0.06 g chlorocresol, 0.18 g sodium dihydrogen phosphate dehydrate, 0.0012 g concentrated phosphoric acid (85%) and 41.488 g distilled water, available commercial under the brand name Essex Cream (Schering AG, Germany), was added to the ALA-containing solution and stirred for 15 minutes.

The composition was divided into four parts and the pH of the subparts of the composition was adjusted to the following four pHs: 1.78, 2.40, 2.94 and 4.03. The formulation was stored at an elevated temperature of 50°C and the decomposition checked in intervals of two and four weeks by measuring the ALA content. The results of this test are set forth in Table 1 below and show that the compositions of this invention retain the active ingredient in high levels, even after 16 weeks of storage:

**Table 1**

| ALA content dependent on pH (stability test) | | | | |
|---|---|---|---|---|
| Time | pH 1.78 | pH 2.40 | pH 2.94 | pH 4.03 |
| 0 week | 100 % | 100 % | 100 % | 100 % |
| 2 weeks | 95.8 % | 99.9 % | 95.1 % | - |
| 6 weeks | 98.1 % | 101.7 % | 96.0 % | 51.8 % |
| 10 weeks | 97.9 % | 105.2 % | 89.7 % | 43.8 |
| | | | | 13 weeks |
| 16 weeks | 98.7 % | 101.7 % | 93.6 % | - |

### Example 2

A cream was prepared in accordance with the procedure set forth in Example 1, the composition was separated into four parts and the pH of each adjusted as follows: pH 1.8; pH 2.4; pH 2.9 and pH 3. The composition having pH 3 was further divided into two parts and sodium lauryl sulfonate (SLS) introduced to final content in the first of 0.15% and in the second, 0.30%. These creams were used to determine the primary dermal skin irritation in a New Zealand white rabbit modified Draize PDI-test (intact and abraded sites for 24 and 72 hours). This Primary Dermal Irritation test was performed in accordance with The International Organization for Standardization Part 10: Test for irritation and sensitization; also described in the British Standard: Evaluation of Medical Devices for Biological Hazards: Part 7: Method of Test for skin irritation of extracts from Medical Devices). A hairless albino guinea pig accumulative irritation test was also performed for 24, 48, 72 and 96 hours to determine the accumulated skin irritation. In this test, the composition was placed on the hairless albino guinea pigs under occlusion for four hours a day for four consecutive days and the reactions noted. The results of these tests are set forth in Table 2 below. It can be seen from the table that the acidic creams of this invention are sufficiently tolerated by the skin, even though the pH of the creams is quite low.

**Table 2**

| **Primary Dermal Irritation and Cumulative Irritation** | | |
|---|---|---|
| **Formula #** | **Primary Deram Irritation** | **Cumulative Skin Irritation** |
| 1A, pH 1.8 | 2.0, Mild irritant | 2.75 |
| 1B, pH 2.9 | 2.0, Mild irritant | 2.67 |
| 1C, pH 2.4 | 1.3, Mild irritant | 2.75 |
| 2A, pH 3, with 0.15% SLS | 2.1, Mild-moderate irritant | (No data phase separation) |
| 2B, pH 3, with 0.30% SLS | 2.0, Mild irritant | 1.83 |

### Example 3

A composition was prepared in accordance with the procedure set forth in Svanberg, et al. containing 9.00% petrolatum, 3.60% mineral oil, 4.32% cetostearyl alcohol, 1.35% cetomacrogol 1000, 0.06% chlorocresol, 0.18% sodium dihydrogen phosphate dihydrate, 0.0012% phosphoric acid, 20% ALA hydrochloride and 61.48% purified water. Another composition was prepared in accordance with the teachings of this invention containing 20% 5-aminolaevulinic acid hydrochloride, 0.20% sodium EDTA and purified water q.s. 100%. The pH of this solution was adjusted to 3 using 50% NaOH.

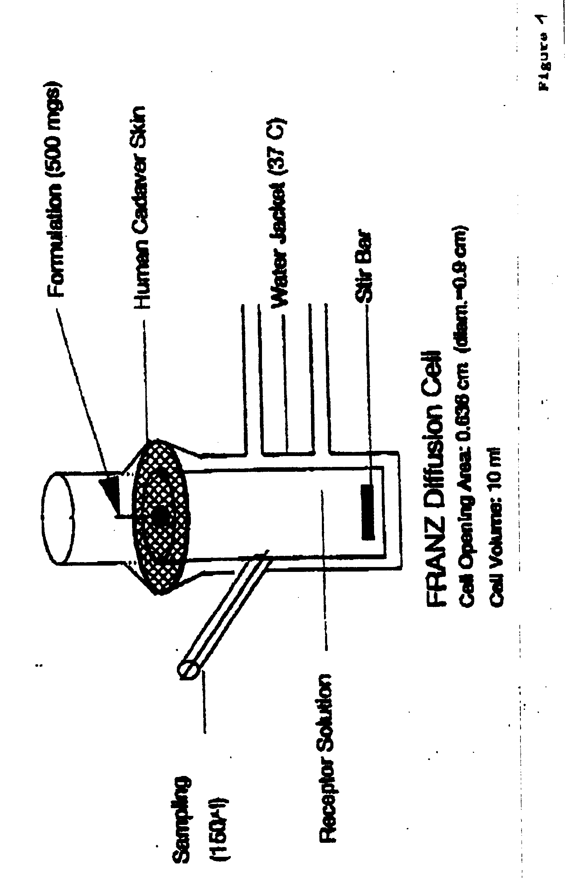

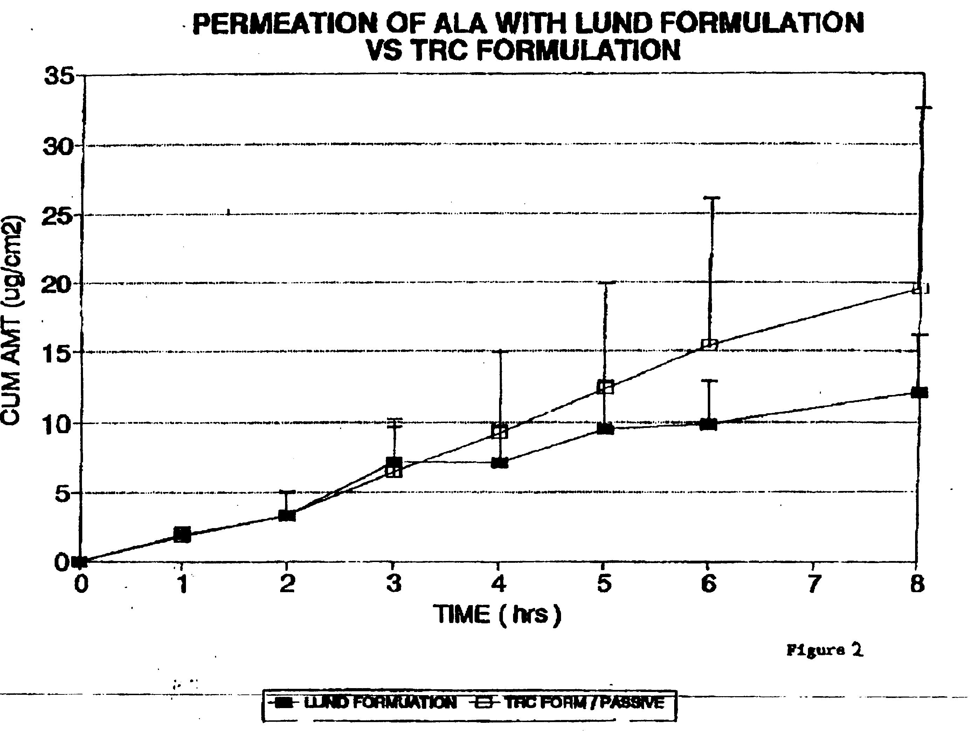

Using these two formulations, penetration studies were conducted on human cadaver skin using the equipment illustrated in Figure 1. The formulation was placed in the upper portion of the Franz diffusion cell depicted in Figure 1. The penetration of the active ingredient was measured by determining the concentration of active ingredient present in the receptor solution. The uptake of active ingredient over time was measured and plotted as a graph, a representation of which is set forth in Figure 2. As can be seen by examination of Figure 2, both formulations penetrate well into and through the skin. The solution having pH 3 exhibits penetration enhanced by a factor of about 1.5 with respect to the Svanberg formulation.

### Example 4

An in vitro skin penetration study was performed comparing the penetration of the following compositions: (1) a formulation containing ALA according to WO 91/01727 (20% ALA in Glaxal base); (2) a water-in-oil emulsion cream (20% ALA in Eucerin); (3) the Svanberg formulation of Example 3, adjusted to a pH of 1.73 and (4) the following formulation in accordance with the teachings of this invention, adjusted to a pH of 3.0 using 50% NaOH: 9.00% petrolatum, 3.60% mineral oil, 4.32% cetostearyl alcohol, 1.35% cetomacrogol 1000, 0.06% chlorocresol, 0.18% sodium dihydrogen phosphate dihydrate, 0.0012% phosphoric acid, 20.0% 5-aminolaevulinic acid and purified water. Both compositions (3) and (4) are oil-in-water compositions. The study was performed using the Franz diffusion cell as set forth in Examples 3 and 4. The results of this study are set forth in Table 3, below. It can been seen that composition (4) clearly has an enhanced permeation through human cadaver skin and is storage-stable.

**TABLE 3**

| **Formulation Stability weeks** | **Amount permetated** | |
|---|---|---|
| | **(µg/cm)** | **50°C/8** |
| (1) | 50.48 | |
| 100.69% | | |
| (2) | 0 | |
| 100.69% | | |
| (3) | 115.73 | 93.26% |
| (4) | 342.74 | |
| 95.31%(16wks.) | | |

## Claims

1. A transdermally acting pharmaceutical ready-to-use composition for the treatment of superficial cytotoxically sensitive disorders, comprising a compound selected from the group of 5-aminolaevulinic acid and derivatives thereof, said composition being capable of topical application to skin, being physically and chemically stable, being bioavailable and having a pH from about 1.8 to about 4.5.

2. A composition according to claim 1 wherein said pH is from about 1.8 to about 3.6.

3. A composition according to claim 1, wherein said composition comprises an oil-in-water emulsion comprising an aliphatic hydrocarbon.

4. A composition according to claim 3, wherein said composition comprises an ointment.

5. A composition according to claim 3, wherein said composition comprises a cream.

6. A composition according to claim 3, wherein said aliphatic hydrocarbon is paraffin.

7. A composition according to claim 6, wherein said paraffin is selected from the group consisting of vaseline, paraffin liquidum and a mixture thereof.

8. A composition according to claim 1 wherein said composition further comprises an emulsifier, a thickening agent, a penetration enhancer and a surfactant.

9. A composition according to claim 3 wherein said active ingredient is present in the amount of from about 2 to about 40% by weight of the composition.

10. A process for preparing a pharmaceutical composition according to claim 8 comprising mixing from about 2 to about 40% 5-aminolaevulinic acid, from about 5 to about 40% of oil, from about 0 to about 7% of an emulsifier, from about 0 to about 1% of a preservative intimately and adjusting the pH to from about 0.9 to about 4.5.
